# EUROPÄISCHE PATENTSCHRIFT

(11) **EP 2 913 072 B1**
(45) Veröffentlichungstag und Bekanntmachung des Hinweises auf die Patenterteilung: **17.04.2019**
(21) Anmeldenummer: 15154745.2
(22) Anmeldetag: 11.02.2015
(51) Int. Cl.: A61M 1/36

(54) **System zur Erkennung einer venösen Nadeldiskonnektion**
System for detecting a venous needle disconnection
Système destinés à la reconnaissance d'une déconnexion d'aiguille veineuse

(30) Priorität: 28.02.2014 DE 102014102732
(43) Veröffentlichungstag der Anmeldung: 02.09.2015
(73) Patentinhaber: B. Braun Avitum AG, 34212 Melsungen (DE)
(72) Erfinder: Steger, Jennifer, 34212 Melsungen (DE)
(74) Vertreter: Winter, Brandl, Fürniss, Hübner, Röss, Kaiser, Polte - Partnerschaft mbB

(56) Entgegenhaltungen:
- EP-A1- 2 218 470
- DE-A1-102008 059 379
- US-A1- 2003 128 125
- US-A1- 2008 065 006
- US-A1- 2010 022 934

## Beschreibung

Die Erfindung betrifft ein System zur Erkennung einer venösen Nadeldiskonnektion, insbesondere bei einer extrakorporalen Blutbehandlung. Die extrakorporale Blutbehandlung kann bei einer Durchführung einer Blutbehandlungstherapie wie z.B. einer Hämodialyse, Hämofiltration oder einer Hämodiafiltration eingesetzt werden. Hierbei wird in der Regel eine Vorrichtung zur Überwachung eines venösen Gefäßzugangs während der extrakorporalen Blutbehandlung eingesetzt.

Zur Blutbehandlung kann das Blut eines Patienten beispielsweise im Zuge einer Hämodialyse, Hämofiltration oder Hämodiafiltration über einen extrakorporalen Blutkreislauf geleitet werden. Um Zugang zum Blutgefäßsystem des Patienten zu erhalten, können arteriovenöse Fisteln, Shunts oder auch Gefäßimplantate verwendet werden. Die Verbindung des extrakorporalen Blutkreislaufs mit dem Patienten erfolgt üblicherweise über Katheter oder Kanülen bzw. Nadeln, z.B. Dialysekanülen oder - nadeln, mit denen beispielsweise eine Fistel oder ein Shunt bzw. ein Gefäßimplantat punktiert wird und hierdurch eine Fluidverbindung hergestellt wird.

Bei Beginn oder auch während einer Blutbehandlung kann der Fall auftreten, dass der venöse Zugang zum Blutkreislauf gestört wird, wenn beispielsweise die Nadel oder Kanüle verrutscht und der extrakorporale Kreislauf nicht mehr ordnungsgemäß, oder überhaupt nicht mehr, mit dem intrakorporalen Blutkreislauf, auch Patientenblutkreislauf, verbunden ist. Dies kann insbesondere bei einer Diskonnektion des venösen Zugangs zum Patientenblutkreislauf problematisch werden. Wird eine solche Diskonnektion des venösen Zugangs nicht rechtzeitig erkannt, wird über den arteriellen Zugang weiterhin Blut aus dem Patienten entnommen, aber nicht mehr ordnungsgemäß in den Patientenkörper nach der extrakorporalen Blutbehandlung zurückgeleitet. Bei üblichen Blutflussraten von beispielsweise 300 bis 400 ml/min entwickelt sich innerhalb weniger Minuten eine lebensgefährliche Situation.

In der EP 1 584 339 B1 ist ein Verfahren auf der Basis der Messung von arteriellen und venösen Drücken unter Summen- und Differenzbildung offenbart.

Bei einer Vorrichtung gemäß der US 7648 474 B2 werden die arteriellen und venösen Druckwerte zum Ermitteln einer Nadeldiskonnektion überwacht.

Bei einer Vorrichtung gemäß der DE 10 2008 059 379 A1 zum Messen des Blutdrucks im arteriellen Gefäßzugang werden der arterielle und venöse Gefäßzugang während der Messung abgesperrt, und das Blut wird über einen Bypass stromauf der venösen Absperrung und stromab der arteriellen Absperrung von der venösen Leitung zur arteriellen Leitung zurückgeführt.

Bei einer Vorrichtung gemäß der EP 2 318 073 B1 zum Erkennen einer Nadeldiskonnektion kommt eine Membranblutpumpe zum Einsatz, die abwechselnd einen zeitlichen Strömungsbereich und einen zeitlichen Nichtströmungsbereich aufweist. Während des Nichtströmungsbereichs wird der venöse Druckabfall ausgewertet.

Gemäß der WO 97/10013 und der WO 2011/080187 A1 werden mittels Drucksensoren gemessene, von Pulsgeneratoren erzeugte Druckpulse ausgewertet, um auf einen Unterbrechungsfehler in dem extrakorporalen Blutkreislauf zu schließen.

Gemäß der WO 2012/175267 A1 werden zum Erkennen einer Nadeldiskonnektion Signale eines arteriellen Drucksensors in Bezug auf Frequenzanteile ausgewertet, die mit Druckpulsen von Pulsgeneratoren korrelieren.

Das Detektionsverfahren nach DIN VDE 0753-4 basiert auf der Auswertung des, im extrakorporalen Blutkreislauf gemessenen, venösen Drucks (PV). Hierbei werden abhängig vom aktuellen venösen Druck Grenzen (UCL = Upper Control Limit; LCL = Lower Control Limit) definiert. Bei Unterschreiten der unteren Grenze LCL wird ein venöser Alarm, der mit der Detektion einer venösen Nadeldiskonnektion (VND; Venous Needle Dislodgement) gleichzusetzen ist, ausgelöst.

Aus der Druckschrift EP 2 218 470 A1 ist ein Hämodialysator bekannt, bei dem Fehler während der Behandlung wie beispielsweise ein Lufteinschluss oder ein Blutverlust erfasst werden sollen. Hierdurch sollen selbst kleine Verbindungsfehler detektiert werden. Blutführungsleitungen sind sowohl arteriell als auch venös vorgesehen. Eine Steuereinrichtung schließt eine Klemme, wenn ab dem Start der Vorbereitung eine vorbestimmte Zeitspanne verstrichen ist, wobei eine Fluidführungsleitung entsprechend betreibbar ist, wozu eine Druckvariation in dem Dialysatdruck oder dem venösen Druck für eine bestimmte Zeitdauer ermittelt und mit einem vorbestimmten Referenzwert verglichen wird.

US 2003/0128125 A1 offenbart eine Blutbehandlungsvorrichtung, bei der Blutleckagen verhindert werden sollen. Informationen aus einer Vielzahl von Eingängen werden kombiniert, um die Empfindlichkeit hinsichtlich der Blutleckerfassung zu erhöhen und Falschdetektionen zu verringern.

Aufgabe der Erfindung ist es, ein System zur Erkennung einer venösen Nadeldiskonnektion zu schaffen, das mit hoher Genauigkeit bei fortgesetzter extrakorporaler Blutbehandlung eingesetzt werden kann.

Die Aufgabe wird mit den Merkmalen der unabhängigen Ansprüche gelöst. Die abhängigen Ansprüche sind auf bevorzugte Ausführungsformen der Erfindung gerichtet.

Erfindungsgemäß wird ein System zur Erkennung einer Verbindungsunterbrechung zwischen einer extrakorporalen Blutbehandlungsvorrichtung (Dialysator), insbesondere einer Dialysemaschine, und einem intrakorporalen Patientenblutkreislauf geschaffen, an den die extrakorporale Blutbehandlungsvorrichtung über eine Fluidverbindungseinrichtung vorzugsweise Nadel oder Kanüle, die an einer ersten, venösen Fluidleitung vorzugsweise an deren Leitungsende anbringbar oder angebracht ist und über eine zweite, arterielle Fluidleitung anschließbar oder angeschlossen ist. Insbesondere wird ein System zur Erkennung eines Herausrutschens der Fluidverbindungs- / -ankopplungseinrichtung wie etwa Nadel oder Kanüle aus dem intrakorporalen Patientenblutkreislauf geschaffen (d.h. eine Verbindungsunterbrechung zwischen dem Dialysator und dem Patientenblutkreislauf auf der venösen Seite des extrakorporalen Blutkreislaufs). Das erfindungsgemäße System ist versehen mit einer Leitungsabsperrung zum Absperren (ausschließlich) der ersten, venösen Fluidleitung, die zwischen der Blutbehandlungsvorrichtung und der Fluidverbindungseinrichtung (Nadel, Kanüle) angeordnet ist, einem in der ersten, venösen Fluidleitung und/oder der zweiten, arteriellen Fluidleitung angeordneten Drucksensor zum Messen des in der ersten und/oder zweiten Fluidleitung herrschenden Drucks und einer Steuer- und Auswerteeinrichtung, die unter anderem den zeitlichen Druckverlauf des zumindest während einer Absperrung der ersten Fluidleitung von dem in der venösen Fluidleitung angeordneten Drucksensor gemessenen Drucks auswertet, um hieraus eine Verbindungsunterbrechung oder Verbindungsstörung zwischen der ersten Verbindungseinrichtung und dem Patientenblutkreislauf zu erkennen, wobei die Steuer- und Auswerteeinrichtung eine für den Druckverlauf repräsentative Größe aus dem Druckverlauf bestimmt, die repräsentative Größe mit einem Referenzwert vergleicht, und entsprechend dem Ergebnis des Vergleichs auf ein Vorhandensein oder eine Abwesenheit einer Verbindungsunterbrechung oder Verbindungsstörung zwischen dem intrakorporalen Patientenblutkreislauf und der Blutbehandlungsvorrichtung schließt. Vorzugsweise führt das erfindungsgemäße System die Verbindungsunterbrechnungs-Erkennung und damit den Absperrvorgang der venösen Fluidleitung während der Patientenbehandlung durch, ohne diese unterbrechen zu müssen.

Das System weist außerdem eine Blutpumpe auf, wobei der Druck für die Auswertung des Druckverlaufs während eines Blutförderbetriebs der Blutpumpe (vorzugsweise ohne Unterbrechung des an der Behandlungsvorrichtung eingestellten Patientenbehandlungsmodus) gemessen wird.

Die erste Fluidleitung (venös) kann in regelmäßigen oder unregelmäßigen Zeitabständen abgesperrt und geöffnet werden.

Gemäß einer Ausführungsform der Erfindung ist der Drucksensor in der ersten (venösen) Fluidleitung zwischen der Leitungsabsperrung und der ersten Verbindungseinrichtung angeordnet, wobei die repräsentative Größe ein Mittelwert, Median, Minimum oder Maximum des Druckverlaufs ist, der Referenzwert ein Mittelwert, Median, Minimum oder Maximum des Druckverlaufs bei einer nicht vorhandenen Verbindungsunterbrechung oder Verbindungsstörung zwischen der ersten Verbindungseinrichtung und dem Patientenblutkreislauf ist, und die Steuer- und Auswerteeinrichtung auf die Verbindungsunterbrechung oder Verbindungsstörung schließt, wenn die repräsentative Größe um mehr als einen vorbestimmten Betrag von dem Referenzwert abweicht.

Der Referenzwert ist dabei vorzugsweise die repräsentative Größe eines vorherigen Druckverlaufs des von dem Drucksensor während einer vorherigen Absperrung der Fluidleitung gemessenen Drucks.

Gemäß einer weiteren Ausführungsform der Erfindung ist der Drucksensor in der ersten Fluidleitung zwischen der Leitungsabsperrung und der ersten Verbindungseinrichtung angeordnet, und die Steuer- und Auswerteeinrichtung bestimmt eine angenäherte Polynomfunktion, insbesondere 1. oder 2. Ordnung, des Druckverlaufs, bestimmt ein Streuungsmaß, insbesondere Varianz oder mittlerer Fehler, aus Abweichungen einzelner Werte des Druckverlaufs von der angenäherten Polynomfunktion als die repräsentative Größe, und schließt auf die Verbindungsunterbrechung oder Verbindungsstörung, wenn die repräsentative Größe um nicht mehr als einen vorbestimmten Betrag von dem Referenzwert abweicht.

Dabei wird vorzugsweise ein vorbestimmter Zeitabschnitt des Druckverlaufs zu Beginn der Absperrung der ersten Fluidleitung bei der Auswertung nicht berücksichtigt.

Gemäß einer weiteren Ausführungsform der Erfindung ist der Drucksensor in der zweiten Fluidleitung angeordnet, und die Steuer- und Auswerteeinrichtung bestimmt ein Frequenzspektrum des Druckverlaufs, wobei die repräsentative Größe eine Amplitude, insbesondere maximale Amplitude, eines vorbestimmten Frequenzbereichs des Frequenzspektrums ist, die mit einer Absperrfrequenz der ersten Fluidleitung korrespondiert, und schließt auf die Verbindungsunterbrechung oder Verbindungsstörung, wenn die repräsentative Größe um nicht mehr als einen vorbestimmten Betrag von dem Referenzwert abweicht.

Erfindungsgemäß ist es möglich, sogenannte venöse Nadeldiskonnektionen (VND) oder Dislokationen zu erkennen. Beispielsweise kann aufgrund von Bewegungen des Patienten oder einer unzureichenden Befestigung der Nadel ein Verrutschen bis hin zum gänzlichen Herausrutschen der Nadel aus der Einstichposition auftreten. Dies ist insbesondere im Bereich der venösen Nadel problematisch, da über diese das Blut zu dem Patienten zurückgeführt wird, wobei das Blut hier unter entsprechendem Druck steht. Dies kann die Gefahr einer unerwünschten Verlagerung bis hin zu einem Herausrutschen der Nadel noch weiter erhöhen.

Im vorliegenden Zusammenhang ist unter dem Ausdruck Nadeldiskonnektion sowohl ein vollständiges Herausrutschen der Nadel aus dem venösen Gefäßzugang als auch ein nur teilweises Dislozieren der Nadel mit eingeschränkter, aber noch in gewissem Umfang vorhandener Verbindung zum Blutkreislauf zu verstehen. Bei nur partiellem Verrutschen der Nadel kann die Nadel in dem die eigentliche Zugangsstelle umgebenden Gewebe liegen und in diesem eine paravasale Blutung hervorrufen.

Im Folgenden wird als "venöser Zweig des extrakorporalen Kreislaufs" eine Blutführung, z.B. ein System oder eine Anordnung bezeichnet, bei der ein einem Gegenstand oder einem Patienten entnommenes Fluid, z.B. Blut, außerhalb des Patientenkörpers nach Durchlaufen einer Fluidbehandlungsvorrichtung in einem Nebenkreislauf geführt und anschließend dem intrakorporalen Patientenkreislauf wieder zugeführt wird. Als "arterieller Zweig des extrakorporalen Kreislaufs" ist ein System oder eine Anordnung zu verstehen, welche ein FLuid (Blut) aus dem Patientenkörper entnimmt und der Fluidbehandlungsvorrichtung zuführt. Der venöse Zweig des extrakorporalen Kreislaufs kann beispielsweise einen das Fluid, das dem Gegenstand oder Patienten entnommen und zu einer Behandlungseinrichtung, wie etwa einer Reinigungseinrichtung oder einem Dialysator zugeführt wurde, von der Behandlungseinrichtung zum Gegenstand oder Patienten zurückführenden, also das Fluid abführenden Schlauch, der auch als Blutabführleitung des Dialysators ausgebildet sein kann, aufweisen.

Weiterhin erfindungsgemäß ist nicht nur die Erkennung einer ungenügenden Verbindung einer Nadel/Kanüle wie etwa einer venösen Nadel bis hin zum Herausrutschen der Nadel aus dem vorgesehenen Zugangsort am Patienten, sondern auch das Erfassen z.B. eines Blutlecks im venösen Zweig hinter der Vorrichtung zur Erkennung einer Verbindungsunterbrechung möglich in Abhängigkeit von der Größe des Blutlecks und/oder der Empfindlichkeit des Sensors.

Die Erfindung wird nachstehend unter Bezugnahme auf die Zeichnungen und bevorzugte Ausführungsformen näher erläutert. Es zeigen:
Fig. 1 eine Ausführungsform eines erfindungsgemäßen Systems;
Fig. 2 ein Diagramm der zeitlichen Druckverläufe des arteriellen Drucks (a), des Shuntdrucks (b) und des venösen Drucks (c) sowie des zeitlichen Klemmenschließ- und öffnungsverlaufs (d) vor (Konnektion) und nach (Diskonnektion) einer Nadeldiskonnektion;
Fig. 3 einen vergrößerten Ausschnitt aus dem Diagramm der Fig. 2;
Fig. 4 ein Diagramm des zeitlichen venösen Druckverlaufs vor und nach einer Bewegung des Patienten;
Fig. 5 ein Diagramm des zeitlichen venösen Druckverlaufs vor und nach einer Nadeldiskonnektion;
Fig. 6 ein Diagramm des Endniveaus des venösen Drucks für verschiedene Shuntdrücke bei Diskonnektion und bei Konnektion;
Fig. 7 ein Diagramm des mittleren Fehlers mit seiner der Standardabweichung von einer Annäherungsfunktion des venösen Druckverlaufs bei Konnektion (1) und bei Diskonnektion (2); und
Fig. 8 ein Frequenzspektrum des arteriellen Drucks für Konnektion und Diskonnektion.

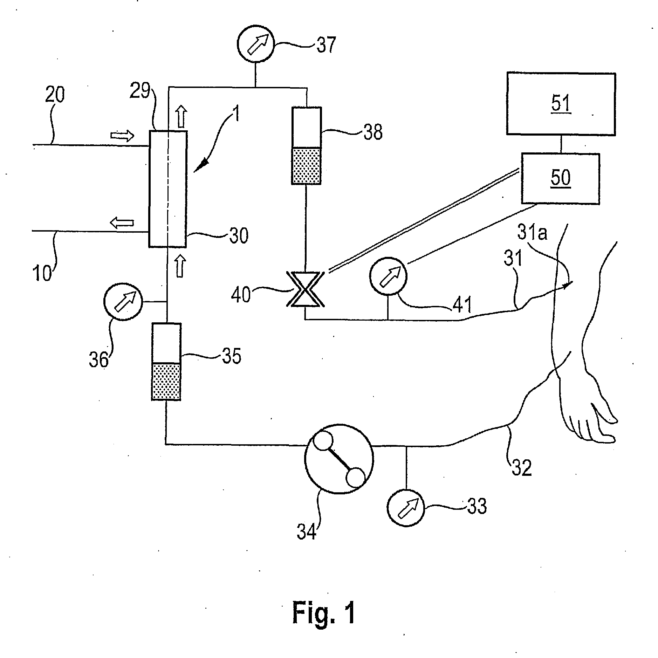

Fig. 1 zeigt eine Ausführungsform eines erfindungsgemäßen Systems zur Erkennung einer venösen Nadeldiskonnektion. Die in Fig. 1 dargestellte Ausführungsform des erfindungsgemäßen Systems zur Erkennung einer fehlerhaften oder fehlenden venösen Verbindung zwischen dem extrakorporalen Blutkreislauf und dem intrakorporalen Patientenblutkreislauf wird beispielhaft anhand einer Dialysemaschine erläutert, welche in der Fig. 1 nur prinzipiell dargestellt ist.

Fig. 1 zeigt demnach einen Dialysator 1, der durch in der Regel eine semipermeable Membran in eine erste, in einem Dialysierflüssigkeitsweg angeordnete Kammer 29 und eine zweite Kammer 30 unterteilt ist, die ihrerseits mittels einer vom Patientenblutkreislauf abführenden (arteriellen) Blutzuführleitung 32 mit dem Dialysatoreinlass verbindbar/verbunden ist. Die zweite Kammer 30 ist mittels eines Dialysatorauslasses mit einer zum Patienten führenden (venösen) Blutabführleitung 31 mit dem Blutkreislauf des Patienten verbindbar/verbunden, um diesem das gereinigte Blut wieder zuzuführen. Die erste Kammer 29 ist mit einem Zulauf 20 für frische Dialysierflüssigkeit und einem Ablauf 10 für verbrauchte Dialysierflüssigkeit verbunden.

Über einen ersten Drucksensor 41 kann der venöse Druck PV nach Abklemmen einer Schlauchsperre (Schlauchabsperrklemme oder Schlauchklemme) 40 oder dergleichen Strömungsabsperrmittel erfasst werden. Die Schlauchklemme 40 kann manuell oder automatisch betätigt werden und eine vollständige oder quasi vollständige Sperrung des Fluidströmungskreislaufs zurück zum venösen Zugang des Patienten bewirken. Bei der Ausführungsform gemäß Fig. 1 ist der Drucksensor 41 für den venösen Druck PV zwischen der die venöse Blutabführleitung 32 absperrende Schlauchklemme 40 und dem venösen Patientenzugang 31a angeordnet und liegt damit im Blutrücklauf zum Patienten. Da der Drucksensor 41 an der Blutabführleitung 31 unmittelbar vor dem Patienten oder jedenfalls stromab der Schlauchklemme 40 angeordnet ist, stellt sich am Drucksensor 41 bei ordnungsgemäßer Verbindung mit dem Patientenblutkreislauf im wesentlichen der Druck aus dem Patientenzugang ein, wohingegen bei ganz oder teilweise aus dem Patientenzugang herausgetretener Nadel der Atmosphärendruck bzw. der statische Druck (nach einer bestimmten Zeit) gemessen wird.

Wie in Fig. 1 gezeigt, sind im arteriellen Zweig bzw. der Blutzuführleitung 32 des extrakorporalen Blutkreislaufs, d.h. in dem von dem Patienten zum Dialysatoreingang führenden Zweig, ein Drucksensor 33 zum Erfassen des arteriellen Drucks PA und ein Blasenfänger 35 angeordnet, zwischen denen die Blutpumpe 34 angeordnet ist. Ein weiterer Drucksensor 36 erfasst den Druck des Bluts unmittelbar am Dialysatoreinlass des Dialysators 1. Die Dialysierflüssigkeit wird in der durch die Pfeile symbolisierten Richtung über die Dialysierflüssigkeitszuleitung 20 zu der ersten Kammer 29 des Dialysators 1 geleitet und aus dieser über die Dialysierflüssigkeitsabführleitung 10 zum Auslass oder Abfluss geleitet. Über die zweite Kammer 30 des Dialysators 1 strömt das zu reinigende Blut und wird am Dialysatorauslass in Pfeilrichtung zur Blutabführleitung (venöser Zweig) 31 geleitet.

In der Blutabführleitung 31 ist ein weiterer Blasenfänger 38 vorzugsweise vorgesehen. Ein Drucksensor 37 erfasst den venösen Druck PV in der Blutabführleitung 31 am Ausgang des Dialysators 1.

Die Strömungsabsperrung 40, die auch als Schlauchabsperrung oder als Schlauchabsperrklemme ausgeführt sein kann, befindet sich in Blutströmungsrichtung gesehen stromauf des Drucksensors 41, der an oder in der Blutabführleitung 31 angeordnet ist. Die Blutzuführleitung 32 und die Blutabführleitung 31 können beispielsweise als Schläuche ausgebildet sein.

Die Flussrate des Blutkreislaufs (extrakorporal) wird durch die Blutpumpe 34 kontrolliert, wobei an oder in der Blutzuführleitung 32 stromauf (in Strömungsrichtung gesehen) zur Blutpumpe 34 der zweite Drucksensor 33 für die arterielle Druckmessung angeordnet ist.

Wird, wie im vorliegenden bevorzugten Ausführungsbeispiel erfindungsgemäß vorgesehen, die Blutpumpe 34 während der Messung auf venöse Nadeldiskonnektion nicht abgestellt, so ist vorzugsweise im Leitungsbereich zwischen der Blutpumpe 34 und der Schlauchabsperrung 40 ein Reservoir zur Aufnahme des geförderten Bluts vorgesehen. Die Aufnahme des geförderten Bluts kann beispielsweise auch von/zusätzlich zu den Blasenfängern 38 und/oder 35 bewerkstelligt werden.

Nach der Messung auf eventuelle Nadeldiskonnektion kann das im Blasenfänger 38 und/oder 35 zwischengespeicherte Blut nach Öffnung der Schlauchabsperrung 40 wieder an den Patienten zurückgegeben werden, sodass das Speichervolumen der Blasenfängers 38 bei einer nachfolgenden Messung wieder für eine Aufnahme von Blut zur Verfügung steht.

Hierzu kann eine Pegelregulierung in den Speichervolumina der Blasenfänger 38 und/oder 35 vorgesehen sein, sodass der Pegel der in den Blasenfängern 38 und/oder 35 gespeicherten Fluidmenge bei geöffneter Schlauchabsperrung 40 auf einen gewünschten Pegel reguliert wird.

Eine Steuereinrichtung, z.B. in Form einer Datenverarbeitungs- und Speichereinheit (Steuer- und Auswerteeinrichtung) 50 kontrolliert bzw. steuert einen (notwendigen) Teil oder alle, der in Fig. 1 gezeigten Elemente über geeignete Schnittstellen, wobei in Fig. 1 nur die Verbindung mit dem Drucksensor 41 und der Schlauchabsperrung 40 dargestellt sind. Insbesondere kontrolliert die Datenverarbeitungs- und Speichereinheit 50 auch den arteriellen Drucksensor 33, wenn dieser erfindungsgemäß verwendet wird. Die Datenverarbeitungs- und Speichereinheit 50 sammelt Informationen auch über andere Parameter der Dialysevorrichtung, z.B. Blutfluss, Dialysierflüssigkeitsfluss und/oder Behandlungszeit. Diese Parameter werden zusammen mit den gemessenen Daten verarbeitet. Die Datenverarbeitungs- und Speichereinheit 50 dient zur Verarbeitung der gemessenen Druckwerte sowie zur Steuerung der Blutabsperrung und der Messzeitpunkte. Ferner können eine oder mehrere interne oder externe Speichereinheiten zur Speicherung von Messwerten, berechneten Werten und/oder Zwischenwerten, Ergebnissen etc. sowie der Steuerungsabläufe vorhanden sein.

Die Messwerte von den Drucksensoren 33, 36, 37 und 41 werden über Datenverbindungen, die kabelgebunden und/oder drahtlos ausgebildet sein können, zu der Datenverarbeitungs- und Speichereinheit 50 übertragen, die als Datenverarbeitungs- und Auswerteeinheit ausgebildet ist, um venöse Nadeldiskonnektionen (VND) zu erkennen.

Außerdem ist optional eine Ausgabeeinheit 51 in Form einer Warnsignaleinheit oder Anzeige vorhanden, die über eine kabelgebundene oder drahtlose Datenverbindung mit der Datenverarbeitungs- und Speichereinheit 50 verbunden ist. Die Ausgabeeinheit 51 gibt ein optisches und/oder akustisches Warnsignal und/oder einen Text zur Erklärung eines Problemfalls beispielsweise auf einem Display an, wenn eine Fehlfunktion der Dialysemaschine und/oder der Vorrichtung zur Erkennung einer Nadeldiskonnektion, insbesondere einer venösen Nadeldiskonnektion, erkannt werden und/oder wenn eine Nadeldiskonnektion erkannt wird. Des Weiteren kann die Ausgabeeinheit 51 zur optischen, akustischen oder elektronischen Ausgabe oder einer Ausgabe in anderer Form, beispielsweise in Form elektronischer Speicherung, eines Ausdrucks, einer E-Mailübersendung oder dergleichen, ausgelegt sein.

Auch wenn es in Fig. 1 nicht gezeigt ist, können weiterhin eine oder mehrere nicht dargestellte zusätzliche Messeinrichtungen, Pumpen, Blasenfänger, Luftdetektoren, Absperrklemmen usw. nach Bedarf vorhanden sein.

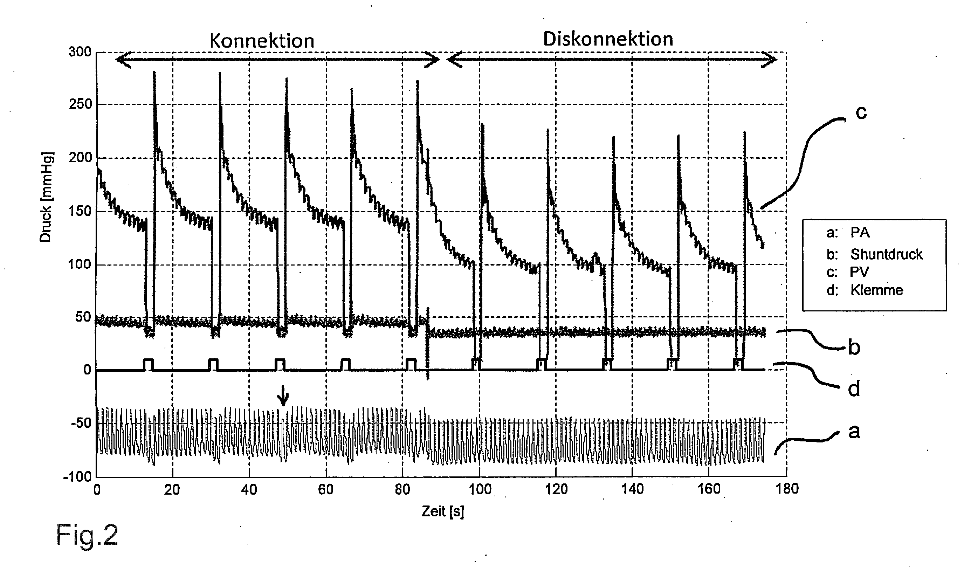

In Fig. 2 ist ein Beispiel der zeitlichen Druckverläufe des von dem (arteriellen) Sensor 33 gemessenen arteriellen Drucks PA (Kurve a), des Shuntdrucks (Kurve b) und des von dem (venösen) Sensor 41 gemessenen venösen Drucks PV (Kurve c) sowie des zeitlichen Klemmenschließ- und öffnungsverlaufs der Absperrung bzw. Klemme 40 (Kurve d) vor und nach einer VND gezeigt. Die Klemme 40 wird gemäß einer bevorzugten Ausführungsform regelmäßig für einen kurzen Zeitraum, beispielsweise 2 Sekunden, geschlossen. Der während dieser Zeit aufgenommene Druck PV des Sensors 41 hängt bei Konnektion von dem Shuntdruck des Patienten ab. Bei Diskonnektion entspricht er dem statischen Druck, der sich durch den Höhenunterschied zwischen Sensor and Kanüle einstellt.

Da die Blutpumpe 34 gemäß einer bevorzugten Ausführungsform und somit auch die Therapie während der Klemmenschließung erfindungsgemäß nicht angehalten werden, das geförderte Blut aber nicht zum Patienten zurückgeführt werden kann, kommt es zu einer Stauung des Blutes in der Tropfkammer 38 und/oder 35, welche sich nach dem Öffnen der Klemme 40 aber wieder entspannt. In der Fig. 2 ist dabei erkennbar, dass entsprechend dem gezeigten Druckverlauf des arteriellen Drucks PA in der arteriellen Blutleitung kein Blut angestaut wird. Ferner lässt sich aus der Fig. 2 deutlich ein Druckverlaufsunterschied im Fall einer Nadelkonnetion zu einer Nadeldiskonnektion am Verlauf der Kurve c erkennen.

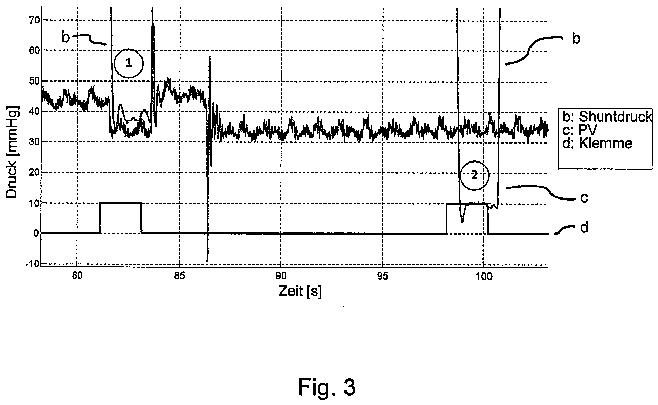

Fig. 2 zeigt demnach einen typischen Druckverlauf bei Verwendung des erfindungsgemäßen Systems für eine beispielhafte Schließzeit von 2 Sekunden und eine beispielhafte Öffnungszeit von 15 Sekunden der Klemme 40 (d.h. einer Klemmenperiode von 17 Sekunden). Es sind jeweils fünf Klemmenschließungen bei Konnektion und Diskonnektion dargestellt. Wie es auch anhand der Fig. 3, die einen Ausschnitt der Fig. 2 zeigt, deutlich erkennbar ist, sinkt der Druck PV, sobald die Klemme 40 geschlossen wird, bei Konnektion (siehe eingekreiste 1 in Fig. 3) auf ein Niveau ab, welches etwa dem Shuntdruck entspricht. Nach dem Öffnen der Klemme 40 entspannt sich das vor der Klemme 40 aufgespannte Volumen, somit steigt der Druck PV schlagartig an und sinkt langsam bis zu einem Gleichgewichtszustand bzw. bis zu dem Zeitpunkt, zu dem die Klemme 40 wieder geschlossen wird. Bei Diskonnektion (siehe eingekreiste 2 in Fig. 3) sinkt der Druck PV jedoch auf ein niedrigeres Niveau ab, das dem statischen Druck entspricht. Aus einer Differenz zwischen den beiden Niveaus kann demnach auf eine VND geschlossen werden.

Gemäß einer ersten erfindungsgemäßen Ausführungsform wird somit ein Wert w (z. B. Mittelwert, Median, Minimum, Maximum etc.) des Druckverlaufs von PV über eine Schließzeit der Klemme (z. B. 2 Sekunden) gebildet, der das Niveau von PV repräsentiert. Der zu Beginn der Messungen oder zu Beginn der Behandlung ermittelte Wert w wird beispielsweise als ein Referenzwert Rw verwendet. Der Referenzwert Rw kann jedoch auch jedes Mal neu aus dem derzeitigen aktuellen Wert w oder aus einem anderen vorherigen Wert w gebildet (aktualisiert) werden und als Referenzwert Rw für den Wert w der nächsten Klemmschließung verwendet werden. Bei jeder neuen oder anschließend an jede neue Klemmenschließung wird der aktuelle Wert w mit dem Referenzwert Rw verglichen. Es wird beispielsweise eine Differenz Δw zwischen dem aktuellen Wert w und dem Referenzwert Rw, vorzugsweise Δw = |w - Rw|, berechnet. Wenn die Differenz Δw einen vorbestimmten (ersten) Schwellenwert oder Grenzwert überschreitet, wird auf eine VND geschlossen. Anders gesagt, wird auf eine VND geschlossen, wenn der aktuelle Wert w von dem Referenzwert Rw um mehr als einen vorbestimmten Betrag abweicht.

Bei der Fortsetzung des Verfahrens nach der Erkennung einer VND (ohne erfolgte Beseitigung) wird vorzugsweise, wenn die Differenz Δw den vorbestimmten Schwellenwert oder Grenzwert überschreitet und der Referenzwert Rw jeweils wie oben beschrieben aktualisiert wird, der Wert w, der um mehr als den vorbestimmten Betrag von dem Referenzwert Rw abweicht, nicht zur Aktualisierung des Referenzwerts Rw verwendet. Stattdessen kann der aktuelle Referenzwert oder ein anderer vorheriger Referenzwert als neuer Referenzwert verwendet werden.

Mittels einer Fortsetzung des Verfahrens, auch wenn eine VND erkannt wurde, kann beispielsweise anhand eines oder mehrerer weiterer Werte w anschließender Klemmenschließungen das Ergebnis der Erkennung einer VND bestätigt oder als fehlerhaft bestimmt werden.

Es kann aber auch der Wert w, der um mehr als den vorbestimmten Betrag von dem Referenzwert Rw abweicht, stattdessen oder zusätzlich als ein weiterer Referenzwert Rw' bei einer Fortsetzung des Verfahrens verwendet werden. Wenn dann der nächste Wert w mit dem neuen Referenzwert Rw' verglichen wird und der Absolutwert der Differenz Δw' = w - Rw' zwischen den beiden einen zweiten Schwellenwert oder Grenzwert, der kleiner als der erste Schwellenwert bzw. Grenzwert ist, nicht überschreitet, kann die Erkennung der VND bestätigt werden. Ansonsten kann beispielsweise auf einen Fehler der Erkennung der VND geschlossen werden, der beispielsweise von einer Patientenbewegung herrühren kann.

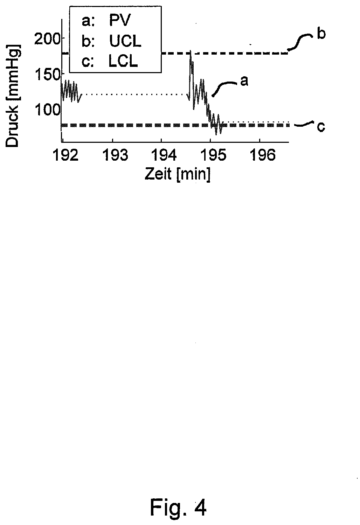

Der venöse Druck kann während einer Therapie aufgrund von Patientenbewegungen stark schwanken. Patientenbewegungen, die den venösen Druck beeinflussen, können das Heben oder Senken des punktierten Arms oder auch das Hinlegen und Aufsetzen eines Patienten sein. Fig. 4 zeigt ein Beispiel für eine venöse Druckschwankung, die auf eine Bewegung des Patienten zurückzuführen ist. In dem Beispiel beträgt die Druckdifferenz bzw. Druckänderung ca. 40mmHg, was einer vertikalen Lageänderung von 50cm entspricht.

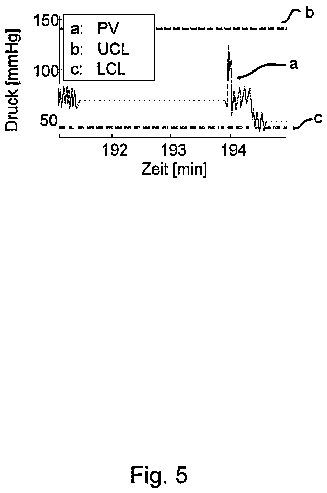

Bei einer VND verringert sich der venöse Druck um den Shuntdruck. Der physiologische Shuntdruck liegt etwa zwischen 15 und 45mmHg, somit kann etwa eine maximale Druckdifferenz von 45mmHg erwartet werden. In Fig. 5 ist beispielhaft der Verlauf eines venösen Drucks mit einer Druckänderung aufgrund einer VND gezeigt. Der Druckabfall beträgt dort ca. 25mmHg.

Die Daten, auf denen die Diagramme der Figuren 4 und 5 beruhen, wurden durch Experimente an einem Versuchsstand, der den menschlichen Shuntkreislauf nachbildet, ermittelt. Es wurde Wasser als Blutersatzsstoff verwendet. Die eingezeichneten oberen und unteren Grenzen UCL und LCL (DIN VDE 0753-4) entsprechen den während der Therapie durch die Dialysemaschine berechneten Grenzen.

Aus den Figuren 4 und 5 wird deutlich, dass die Detektion einer VND (Druckabfall < 45mmHg), aufgrund der Druckschwankungen während einer Dialysetherapie eine Herausforderung darstellt. In der Praxis kommt es häufig zu Falschalarmen aufgrund von Patientenbewegungen (oder auch Änderungen von Parametern wie Ultrafiltrationsrate UFR, Dialysierflüssigkeitsfluss DF oder Levelregulierung LR, die den venösen Druck beeinflussen, aber beispielsweise bei der Berechnung der Grenzen UCL und LCL nicht berücksichtigt werden). Außerdem werden VNDs oft erst spät oder gar nicht erkannt, da die untere Grenze LCL aufgrund einer zu geringen Druckdifferenz nicht unterschritten wird.

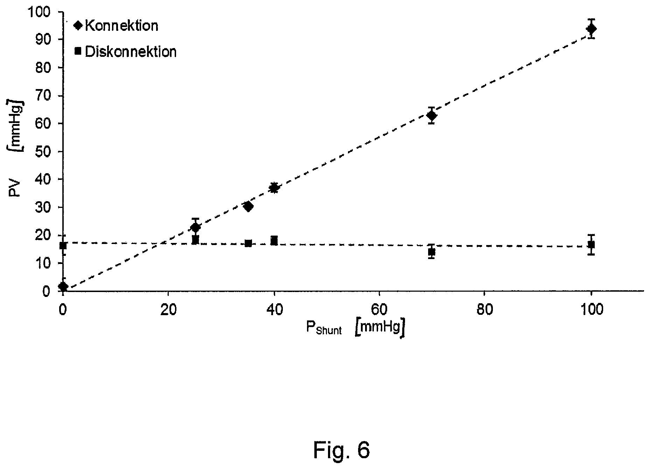

Der verbleibende statische Druck bei Diskonnektion, der von der Position von Sensor und Kanüle abhängig ist, liegt bei etwa 10 bis 20mmHg (es sind auch andere Druckwerte möglich, falls die Kanüle zu Boden fällt). Wie es aus der Figur 6 ersichtlich ist, die ein Diagramm des Endniveaus des venösen Drucks für verschiedene Shuntdrücke jeweils für Diskonnektion und Konnektion zeigt, kann eine klare Unterscheidung zwischen Konnektion und Diskonnektion für Patienten mit kleinen Shuntdrücken schon ab einem Shuntdruck von 25mmHg (und niedriger) auch mit dem obigen Verfahren gemäß der ersten Ausführungsform schwierig werden.

Bei einem Vergleich der venösen Druckverläufe PV bei geschlossener Klemme bei Konnektion und bei Diskonnektion (1 = Konnektion; 2 = Diskonnektion) in Fig. 3 fällt auf, dass sie sich nicht nur in ihrem Niveau, sondern auch in ihrem Verlauf unterscheiden. Beide Druckverläufe bei geschlossener Klemme weisen ein erstes "systembedingtes" Minimum auf, welches aufgrund des plötzlichen Druckabfalls (Ausströmen des Bluts aus der Kanüle) erzeugt wird und abhängig von den Bluteigenschaften (z. B. Viskosität) und dem Kanülendurchmesser ist. Nach Abklingen dieses systembedingten Minimums beschreibt der Verlauf bei Diskonnektion (2) daraufhin angenähert eine Gerade oder leicht gekrümmte Kurve, wohingegen der Verlauf bei Konnektion (1) eine geringe Pulsation bzw. Schwingung aufweist. Diese Pulsation wird durch die Blutpumpe verursacht. Die durch die Blutpumpe erzeugte Pulsation des arteriellen Drucks überträgt sich über den arteriellen Patientenanschluss auf den Shuntdruck (Patienten) und von dort aus über den venösen Patientenanschluss auf den venösen Druck PV. Somit ist die Pulsation, stark gedämpft, auch in dem Druckverlauf von PV zu erkennen. Diese Pulsation kann unabhängig von Patientenbewegungen detektiert werden, da das Niveau (Gleichanteil) des Druckes PV selbst irrelevant für die Detektion ist.

Gemäß einer zweiten Ausführungsform wird daher der Druckverlauf bei geschlossener Klemme durch eine Polynomfunktion, vorzugsweise 1. oder 2. Ordnung angenähert. Dieses kann beispielsweise durch eine Polynomregression (entsprechend 1. oder 2. Grades) erfolgen.

Anschließend wird beispielsweise eine Varianz oder ein mittlerer Fehler aus den Abweichungen der verwendeten Druckwerte von der angenäherten Polynomfunktion ermittelt, indem ein Mittelwert der Abweichungen berechnet wird. Alternativ kann ein mittlerer quadratischer Fehler oder einer mittlerer Fehler höherer Ordnung aus den Abweichungen berechnet werden.

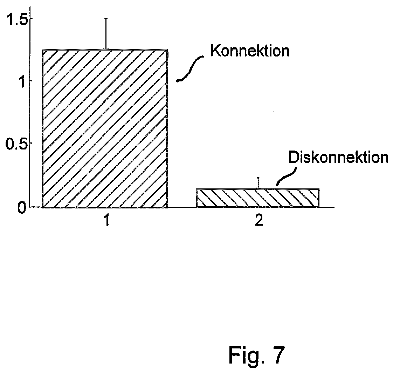

Fig. 7 zeigt beispielhaft einen mittleren Fehler für den PV-Druckverlauf für Konnektion (1) und Diskonnektion (2) und die zughörige Standardabweichung (vertikale Linie). Der mittlere Fehler bei Konnektion beträgt hier etwa 1,25 und der mittlere Fehler bei Diskonnektion beträgt nur etwa 0,2. Hieraus wird deutlich, dass der mittlere Fehler bei Konnektion im Vergleich zum mittleren Fehler bei Diskonnektion deutlich größer ist. Dies liegt daran, dass eine Polynomfunktion 1. oder 2. Ordnung den PV-Druckverlauf bei geschlossener Klemme bei Diskonnektion gut annähert, bei Konnektion aber nicht.

Der wie oben berechnete Fehler, beispielsweise mittlere Fehler, wird dann mit einem Schwellenwert bzw. Grenzwert verglichen. Wenn der Fehler kleiner als der Schwellenwert ist, wird auf eine VND geschlossen. Der Schwellenwert kann im obigen Fall der Fig. 7 beispielsweise 0,3 betragen. Andere sinnvolle Werte, die beispielsweise geeignet durch Experimente und/oder Simulationen ermittelt werden, und mittels denen zuverlässig zwischen dem obigen berechneten Fehler der PV-Druckwerte bei Konnektion und bei Diskonnektion unterschieden werden kann, sind auch möglich.

Zur Berechnung der Polynomfunktion wird vorzugsweise das oben angesprochene systembedingte Minimum außer Acht gelassen. Hierfür werden beispielsweise die Werte des PV-Druckverlaufs zu Beginn bei geschlossener Klemme, d.h. der ersten Sekunden, z.B. 0,6 Sekunden, nicht verwendet. Dieser Zeitraum zu Beginn (ebenfalls abhängig vom Blutfluss und der gewählten Nadel-/Kanülengröße) kann in Abhängigkeit von der Gesamtklemmenschließzeit und/oder der Klemmenschließ/-Öffnungsperiode bestimmt werden.

Es wird nun wieder Fig. 2, insbesondere der dort gezeigte Verlauf des arteriellen Drucks PA betrachtet. Bei Vergleich des arteriellen Drucks PA vor (Konnektion) und nach (Diskonnektion) der VND (bei ca. 86s) wird deutlich, dass der Druck PA (obere Einhüllende und untere Einhüllende) vor der VND bei jeder Klemmenschließung etwas absinkt und danach wieder ansteigt (siehe Pfeil bei ca. 50s), wohingegen er bei Diskonnektion nahezu konstant ist. Der Effekt des Absinkens wird dadurch hervorgerufen, dass der Shuntfluss aufgrund der Klemmenschließung reduziert ist (Blut wird weiterhin mit ca. 300ml/min entzogen, allerdings nicht wieder zurückgegeben) und somit der Shuntdruck ebenfalls absinkt. Da der arterielle Druck sich, genau wie der venöse Druck, aus dem Shuntdruck und einer Druckkomponente, die durch die Blutpumpe erzeugt wird, zusammensetzt, überträgt sich der Druckabfall des Shuntdrucks auf den arteriellen Druck. Die so erzeugte überlagerte Schwankung bzw. Schwingung macht die Konnektion (Schwankung bzw. Schwingung vorhanden) von der Diskonnektion (Schwankung bzw. Schwingung nicht vorhanden) unterscheidbar.

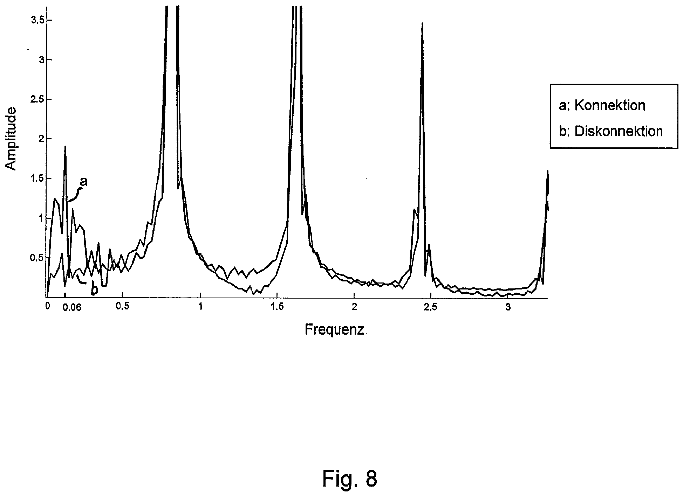

Gemäß einer dritten Ausführungsform wird daher eine Spektralanalyse des für die Zeit der geschlossenen Klemme und eine gewisse darüber hinausgehende Zeitspanne gemessenen PA-Druckverlaufes durchgeführt. Hierfür wird beispielsweise eine schnelle Fouriertransformation FFT durchgeführt. Es sind jedoch auch andere Frequenzanalyseverfahren möglich. Ein Beispiel eines Ergebnisses einer derartigen FFT des PA-Druckverlaufes bei geschlossener Klemme jeweils bei einer Konnektion und einer Diskonnektion ist in Fig. 8 gezeigt.

Das dort gezeigte Hauptmaximum bei 0,8Hz (und seine harmonischen Vielfachen) ist auf die Rotation der Blutpumpe zurückzuführen. Da die Klemme in dem vorliegenden Beispiel alle 17 Sekunden geschlossen wurde, existiert ein zusätzliches Maximum bei ca. 0,06Hz bei Konnektion (Kurve a). Bei Diskonnektion (Kurve b) ist dieses Maximum nicht vorhanden. Somit ist eine klare Unterscheidung zwischen den beiden Zuständen Konnektion und Diskonnektion möglich.

Gemäß der dritten Ausführungsform wird ein Frequenzbereich um die Schließfrequenz fs der Klemme festgelegt, beispielsweise fs ± c x fs (im vorliegenden Beispiel 0,06 Hz ± 0,5 x 0,06 Hz), wobei c eine geeignet gewählte Konstante ist, so dass eine der Auflösung des Frequenzspektrums entsprechende Frequenz der zu erfassenden Schließfrequenz enthalten ist und erfasst werden kann. Dann wird beispielsweise eine maximale Amplitude in diesem Frequenzbereich des FFT-Verlaufes ermittelt. Diese wird dann mit einem Amplitudenschwellenwert bzw. -grenzwert verglichen. Unterschreitet diese maximale Amplitude den Amplitudenschwellenwert, wird auf eine VND geschlossen. Der Amplitudenschwellenwert wird durch aus Experimenten gewonnenen Erfahrungen derart festgelegt, dass eine zuverlässige Unterscheidung zwischen Konnektion und Diskonnektion anhand des Frequenzspektrums möglich ist, d.h. als ein Schwellenwert, den die maximale Amplitude bei Konnektion möglichst sicher überschreitet, der aber von den entsprechenden Amplituden bei Diskonnektion nicht überschritten wird. Der Amplitudenschwellenwert für das hier gezeigte Beispiel beträgt z.B. 1,1.

Die obigen Ausgestaltungen gemäß der ersten, zweiten und dritten Ausführungsform schließen sich nicht gegenseitig aus und können beliebig miteinander kombiniert werden, um die Zuverlässigkeit der Erkennung einer VND zu verbessern. So kann beispielsweise das Ergebnis einer Ausgestaltung (Vorliegen einer VND oder nicht) durch eine andere Ausgestaltung bestätigt oder verworfen werden. Vorteilhaft ist es, wenn die kombinierten Maßnahmen gleichzeitig durchgeführt werden, um so möglichst schnell zu einem zuverlässigen Ergebnis zu kommen. Dies ist möglich, da derselbe Aufbau wie beispielsweise anhand von Fig. 1 beschrieben verwendet werden kann.

Das beschriebene System dient zusammenfassend zur Erkennung einer Verbindungsunterbrechung zwischen einer Blutbehandlungsvorrichtung und einem Patientenblutkreislauf, der über eine erste Verbindungseinrichtung, die an einer ersten Fluidleitung anbringbar mit der Blutbehandlungsvorrichtung in Verbindung bringbar ist, mit einer Leitungsabsperrung zum Absperren der ersten Fluidleitung, die zwischen der Blutbehandlungsvorrichtung und der Verbindungseinrichtung angeordnet ist, einer zwischen der Blutbehandlungsvorrichtung und dem Patientenblutkreislauf angeordneten zweiten Fluidleitung, einem in der ersten Fluidleitung oder der zweiten Fluidleitung angeordneten Drucksensor zum Messen des in der ersten oder zweiten Fluidleitung herrschenden Drucks, und einer Steuer- und Auswerteeinrichtung, die den zeitlichen Druckverlauf des während einer Absperrung der ersten Fluidleitung von dem Drucksensor gemessenen Drucks auswertet, um hieraus eine Verbindungsunterbrechung oder Verbindungsstörung zwischen der ersten Verbindungseinrichtung und dem Patientenblutkreislauf zu erkennen, wobei die Steuer- und Auswerteeinrichtung eine für den Druckverlauf repräsentative Größe aus dem Druckverlauf bestimmt, die repräsentative Größe mit einem Referenzwert vergleicht, und entsprechend dem Ergebnis des Vergleichs auf ein Vorhandensein oder eine Abwesenheit einer Verbindungsunterbrechung oder Verbindungsstörung zwischen der ersten Verbindungseinrichtung und dem Patientenblutkreislauf schließt.

## Patentansprüche

1. System zur Erkennung einer Verbindungsunterbrechung zwischen einer extrakorporalen Blutbehandlungsvorrichtung, vorzugsweise einem Dialysator (1), und einem intrakorporalen Patientenblutkreislauf, an den die extrakorporale Blutbehandlungsvorrichtung (1) über eine Fluidverbindungseinrichtung (31a), vorzugsweise eine Nadel oder Kanüle, die an einer ersten, venösen Fluidleitung (31) vorzugsweise an deren Leitungsende anbringbar oder angebracht ist, und über eine zweite, arterielle Fluidleitung (32) anschließbar oder angeschlossen ist, mit
einer Leitungsabsperrung (40) zum Absperren der ersten, venösen Fluidleitung (31), die zwischen der Blutbehandlungsvorrichtung (1) und der Verbindungseinrichtung (31a) angeordnet ist,
wenigstens einer Blutpumpe (34), die in der zweiten, arteriellen Fluidleitung (32) angeordnet ist und vorzugsweise für eine ununterbrochene Blutförderung auch während des Absperrens der ersten, venösen Fluidleitung (31) durch die Leitungsabsperrung (40) ausgebildet ist,
mindestens einem in der ersten Fluidleitung (31) und/oder der zweiten Fluidleitung (32) angeordneten Drucksensor (33, 41) zum Messen des in der ersten und/oder zweiten Fluidleitung herrschenden Drucks,
einer Steuer- und Auswerteeinrichtung (50), die dazu angepasst ist, den zeitlichen Druckverlauf des während einer Absperrung der ersten Fluidleitung (31) von dem Drucksensor (33, 41) gemessenen Drucks (PA, PV) auszuwerten, um hieraus eine Verbindungsunterbrechung oder Verbindungsstörung zwischen der ersten Fluidverbindungseinrichtung (31a) und dem Patientenblutkreislauf zu erkennen, wofür
die Steuer- und Auswerteeinrichtung (50) dazu ausgelegt ist,
eine für den Druckverlauf repräsentative Größe aus dem Druckverlauf zu bestimmen,
die repräsentative Größe mit einem vorzugsweise vorab gespeicherten Referenzwert oder Referenzwertebereich zu vergleichen, und
entsprechend dem Ergebnis des Vergleichs auf ein Vorhandensein einer Verbindungsunterbrechung oder Verbindungsstörung der ersten venösen Fluidleitung (31) zwischen der Blutbehandlungsvorrichtung (1) und dem Patientenblutkreislauf zu schließen, wobei
der Drucksensor (41) in der ersten Fluidleitung (31) zwischen der Leitungsabsperrung (40) und der Fluidverbindungseinrichtung (31a) angeordnet ist,
**gekennzeichnet dadurch dass**
die Steuer- und Auswerteeinrichtung (50) eine angenäherte Polynomfunktion, insbesondere 1. oder 2. Ordnung, des Druckverlaufs bestimmt,
ein Streuungsmaß, insbesondere Varianz oder mittlerer Fehler, aus Abweichungen einzelner Werte des Druckverlaufs von der angenäherten Polynomfunktion als die repräsentative Größe bestimmt, und
wenn die repräsentative Größe von dem Referenzwert, vorzugsweise um nicht mehr als einen vorbestimmten Betrag, abweicht, insbesondere diesen unterschreitet, auf eine Verbindungsunterbrechung oder Verbindungsstörung schließt.

2. System nach Anspruch 1, wobei die erste Fluidleitung (31) in regelmäßigen oder unregelmäßigen Zeitabständen abgesperrt und geöffnet wird.

3. System nach Anspruch 1, wobei ein vorbestimmter Zeitabschnitt des Druckverlaufs zu Beginn der Absperrung der ersten Fluidleitung (31) bei der Auswertung nicht berücksichtigt wird.

4. System zur Erkennung einer Verbindungsunterbrechung zwischen einer extrakorporalen Blutbehandlungsvorrichtung, vorzugsweise einem Dialysator (1), und einem intrakorporalen Patientenblutkreislauf, an den die extrakorporale Blutbehandlungsvorrichtung (1) über eine Fluidverbindungseinrichtung (31a), vorzugsweise eine Nadel oder Kanüle, die an einer ersten, venösen Fluidleitung (31) vorzugsweise an deren Leitungsende anbringbar oder angebracht ist, und über eine zweite, arterielle Fluidleitung (32) anschließbar oder angeschlossen ist, mit
einer Leitungsabsperrung (40) zum Absperren der ersten, venösen Fluidleitung (31), die zwischen der Blutbehandlungsvorrichtung (1) und der Verbindungseinrichtung (31a) angeordnet ist,
wenigstens einer Blutpumpe (34), die in der zweiten, arteriellen Fluidleitung (32) angeordnet ist und vorzugsweise für eine ununterbrochene Blutförderung auch während des Absperrens der ersten, venösen Fluidleitung (31 durch die Leitungsabsperrung (40) ausgebildet ist,
mindestens einem in der ersten Fluidleitung (31) und/oder der zweiten Fluidleitung (32) angeordneten Drucksensor (33, 41) zum Messen des in der ersten und/oder zweiten Fluidleitung herrschenden Drucks,
einer Steuer- und Auswerteeinrichtung (50), die dazu angepasst ist, den zeitlichen Druckverlauf des während einer Absperrung der ersten Fluidleitung (31) von dem Drucksensor (33, 41) gemessenen Drucks (PA, PV) auszuwerten, um hieraus eine Verbindungsunterbrechung oder Verbindungsstörung zwischen der ersten Fluidverbindungseinrichtung (31a) und dem Patientenblutkreislauf zu erkennen, wofür
die Steuer- und Auswerteeinrichtung (50) dazu ausgelegt ist,
eine für den Druckverlauf repräsentative Größe aus dem Druckverlauf zu bestimmen,
die repräsentative Größe mit einem vorzugsweise vorab gespeicherten Referenzwert oder Referenzwertebereich zu vergleichen, und
entsprechend dem Ergebnis des Vergleichs auf ein Vorhandensein oder eine Abwesenheit einer Verbindungsunterbrechung oder Verbindungsstörung der ersten venösen Fluidleitung (31) zwischen der Blutbehandlungsvorrichtung (1) und dem Patientenblutkreislauf zu schließen, wobei
ein weiterer Drucksensor (33) in der zweiten Fluidleitung (32) angeordnet ist, und **gekennzeichnet dadurch dass**
die Steuer- und Auswerteeinrichtung (50)
ein Frequenzspektrum des Druckverlaufs bestimmt, wobei die repräsentative Größe eine Amplitude, insbesondere maximale Amplitude, eines vorbestimmten Frequenzbereichs des Frequenzspektrums ist, die mit einer Frequenz oder Häufigkeit der Absperrung der ersten Fluidleitung (31) korrespondiert, und
wenn die repräsentative Größe von dem Referenzwert, vorzugsweise um nicht mehr als einen vorbestimmten Betrag, abweicht, insbesondere diesen unterschreitet, auf eine Verbindungsunterbrechung oder Verbindungsstörung schließt.

## Claims

1. A system for detecting an interruption of the connection between an extracorporeal blood treatment apparatus, preferably a dialyser (1), and an intracorporeal patient blood circulation to which the extracorporeal blood treatment apparatus (1) is connectable or connected via a fluid connection means (31a), preferably a needle or cannula, being attachable or attached to a first venous fluid line (31), preferably to the line end thereof, and via a second arterial fluid line (32), comprising
a line shut-off (40) for blocking the first venous fluid line (31) arranged between the blood treatment apparatus (1) and the connection means (31a),
at least one blood pump (34) arranged in the second arterial fluid line (32) and preferably configured for continuous blood delivery also during shut-off of the first venous fluid line (31) by the line shut-off (40),
at least one pressure sensor (33, 41) arranged in the first fluid line (31) and/or the second fluid line (32) for measuring the pressure prevailing in the first and/or second fluid line,
a control and evaluation unit (50) adapted to evaluate the pressure curve in time of the pressure (PA, PV) measured by the pressure sensor (33, 41) during shut-off of the first fluid line (31) so as to detect herefrom an interruption or disturbance of the connection between the first fluid connection means (31a) and the patient blood circulation, for which
the control and evaluation unit (50) is configured to
determine a variable representative of the pressure curve from the pressure curve,
compare the representative variable to a preferably previously stored reference value or range of reference values, and
corresponding to the result of comparison conclude the presence of an interruption or disturbance of the connection of the first venous fluid line (31) between the blood treatment apparatus (1) and the patient circulation, wherein
the pressure sensor (41) is arranged in the first fluid line (31) between the line shut-off (40) and the fluid connection means (31a),
**characterized in that**
the control and evaluation unit (50)
determines an approximated polynomial function, especially of the 1^{st} or 2^{nd} order, of the pressure curve,
determines a measure of scale, especially variance or mean error, from deviations of individual values of the pressure curve from the approximated polynomial function as the representative variable, and
concludes the interruption or disturbance of the connection, if the representative variable deviates from the reference value preferably by no more than a predetermined amount, in particular underruns the same.

2. The system according to claim 1, wherein the first fluid line (31) is blocked and opened at regular or irregular time intervals.

3. The system according to claim 1, wherein a predetermined time interval of the pressure curve at the beginning of shut-off of the first fluid line (31) is not taken into account for evaluation.

4. A system for detecting an interruption of the connection between an extracorporeal blood treatment apparatus, preferably a dialyser (1), and an intracorporeal patient blood circulation to which the extracorporeal blood treatment apparatus (1) is connectable or connected via a fluid connection means (31a), preferably a needle or cannula, being attachable or attached to a first venous fluid line (31), preferably to the line end thereof, and via a second arterial fluid line (32), comprising
a line shut-off (40) for blocking the first venous fluid line (31) arranged between the blood treatment apparatus (1) and the connection means (31a),
at least one blood pump (34) arranged in the second arterial fluid line (32) and preferably configured for continuous blood delivery also during shut-off of the first venous fluid line (31) by the line shut-off (40),
at least one pressure sensor (33, 41) arranged in the first fluid line (31) and/or the second fluid line (32) for measuring the pressure prevailing in the first and/or second fluid line,
a control and evaluation unit (50) adapted to evaluate the pressure curve in time of the pressure (PA, PV) measured by the pressure sensor (33, 41) during shut-off of the first fluid line (31) so as to detect herefrom an interruption or disturbance of the connection between the first fluid connection means (31a) and the patient blood circulation, for which
the control and evaluation unit (50) is configured to
determine a variable representative of the pressure curve from the pressure curve,
compare the representative variable to a preferably previously stored reference value or range of reference values, and
corresponding to the result of comparison conclude the presence of an interruption or disturbance of the connection of the first venous fluid line (31) between the blood treatment apparatus (1) and the patient circulation, wherein
a further pressure sensor (33) is arranged in the second fluid line (32), and **characterized in that**
the control and evaluation unit (50)
determines a frequency spectrum of the pressure curve, the representative variable being an amplitude, especially maximum amplitude, of a predetermined frequency range of the frequency spectrum which corresponds to a shut-off frequency of the first fluid line (31), and
concludes the interruption or disturbance of the connection, if the representative variable deviates from the reference value preferably by no more than a predetermined amount, in particular underruns the same.

## Revendications

1. Système de reconnaissance d'une déconnexion entre un dispositif de traitement de sang extracorporel, de préférence un dialyseur (1), et une circulation sanguine intracorporelle d'un patient, dans laquelle le dispositif de traitement sanguin extracorporel (1) peut être raccordé ou est raccordé via un dispositif de raccordement de fluide (31a), de préférence une aiguille ou une canule, qui peut être appliquée ou est appliquée à un premier conduit de fluide veineux (31), de préférence à son extrémité de conduit, et via un second conduit de fluide artériel (32) présentant
un blocage de conduit (40) pour bloquer le premier conduit veineux (31), qui est agencé entre le dispositif de traitement sanguin (1) et le dispositif de raccordement de fluide (31a),
au moins une hémopompe (34) qui est agencée dans le second conduit de fluide artériel (32) et est conçue de préférence pour un acheminement de sang ininterrompu également au cours du blocage du premier conduit veineux (31) à travers le blocage de conduit (40),
au moins un capteur de tension (33, 41) agencé dans le premier conduit de fluide (31) et/ou le second conduit de fluide (32) pour mesurer la tension régnant dans le premier et/ou le second conduit de fluide,
un dispositif de commande et d'évaluation (50) qui est aménagé pour évaluer l'évolution dans le temps de la tension (PA, PV) mesurée par le capteur de tension (33, 41) au cours d'un blocage du premier conduit de fluide (31) afin de reconnaître ainsi une déconnexion ou une défaillance de connexion entre le premier dispositif de raccordement de fluide (31a) et la circulation sanguine du patient, le dispositif de commande et d'évaluation (50) étant prévu pour
déterminer à partir de l'évolution de la tension une grandeur représentative de l'évolution de la tension,
comparer la grandeur représentative à une valeur de référence ou une plage de valeurs de référence de préférence mémorisée auparavant et
conclure, en fonction du résultat de la comparaison, à une présence d'une déconnexion ou à une défaillance de connexion du premier conduit de fluide veineux (31) entre le dispositif de traitement sanguin (1) et la circulation sanguine du patient, dans lequel
le capteur de tension (41) est agencé dans le premier conduit de fluide (31) entre le blocage de conduit (40) et le dispositif de raccordement de fluide (31a),
**caractérisé en ce que** :
le dispositif de commande et d'évaluation (50)
détermine une fonction polynomiale approchée, en particulier du 1^{er} ou du 2^{nd} ordre, de l'évolution de la tension,
détermine une dispersion, en particulier une variance ou une défaillance moyenne, à partir d'écarts de valeurs individuelles de l'évolution de la tension vis-à-vis de la fonction polynomiale approchée comme grandeur représentative et,
lorsque la grandeur représentative s'écarte de la valeur de référence, de préférence de pas plus d'un montant prédéterminé, en particulier inférieure à celui-ci, conclut à une déconnexion ou à une défaillance de connexion.

2. Système selon la revendication 1, dans lequel le premier conduit de fluide (31) est bloqué et ouvert à intervalles de temps réguliers ou irréguliers.

3. Système selon la revendication 1, dans lequel un intervalle de temps prédéterminé de l'évolution de la tension au début du blocage du premier conduit de fluide (31) n'est pas pris en compte lors de l'évaluation.

4. Système de reconnaissance d'une déconnexion entre un dispositif de traitement sanguin extracorporel, de préférence un dialyseur (1), et une circulation sanguine intracorporelle d'un patient, dans lequel le dispositif de traitement sanguin extracorporel (1) peut être raccordé ou est raccordé via un dispositif de raccordement de fluide (31a), de préférence une aiguille ou une canule, qui peut être appliquée ou est appliquée à un premier conduit de fluide veineux (31), de préférence à son extrémité de conduit, et via un second conduit de fluide artériel (32) présentant
un blocage de conduit (40) pour bloquer le premier conduit veineux (31), qui est agencé entre le dispositif de traitement sanguin (1) et le dispositif de raccordement (31a),
au moins une hémopompe (34) qui est agencée dans le second conduit de fluide artériel (32) et est conçue de préférence pour un acheminement de sang ininterrompu également au cours du blocage du premier conduit de fluide veineux (31) à travers le blocage de conduit (40),
au moins un capteur de tension (33, 41) agencé dans le premier conduit de fluide (31) et/ou le second conduit de fluide (32) pour mesurer la tension régnant dans le premier et/ou le second conduit de fluide,
un dispositif de commande et d'évaluation (50) qui est aménagé pour évaluer l'évolution dans le temps de la tension (PA, PV) mesurée par le capteur de tension (33, 41) au cours d'un blocage du premier conduit de fluide (31) afin de reconnaître ainsi une déconnexion ou une défaillance de connexion entre le premier dispositif de raccordement de fluide (31a) et la circulation sanguine du patient, le dispositif de commande et d'évaluation (50) étant prévu pour
déterminer à partir de l'évolution de la tension une grandeur représentative de l'évolution de la tension,
comparer la grandeur représentative à une valeur de référence ou une plage de valeurs de référence de préférence mémorisée auparavant et
conclure, en fonction du résultat de la comparaison, à une présence ou une absence d'une déconnexion ou d'une défaillance de connexion du premier conduit de fluide veineux (31) entre le dispositif de traitement sanguin (1) et la circulation sanguine du patient, dans lequel
un autre capteur de tension (33) est agencé dans le second conduit de fluide (32), et **caractérisé en ce que**
le dispositif de commande et d'évaluation (50)
détermine un spectre de fréquence de la courbe de tension, dans lequel la grandeur représentative est une amplitude, en particulier une amplitude maximale, d'une plage de fréquences prédéterminée du spectre de fréquence, qui correspond à une fréquence ou à la fréquence du blocage du premier conduit de fluide (31) et,
lorsque la grandeur représentative s'écarte de la valeur de référence, de préférence de pas plus d'un montant prédéterminé, en particulier inférieure à celui-ci, conclut à une déconnexion ou à une défaillance de connexion.
